# FASCICULE DE BREVET EUROPEEN

(11) **EP 3 365 597 B1**
(45) Date de publication et mention de la délivrance du brevet: **03.07.2019**
(21) Numéro de dépôt: 16757697.4
(22) Date de dépôt: 27.07.2016
(51) Int. Cl.: F21V 5/04, F21V 7/00, G02B 19/00, F21Y 115/10

(54) **COLLIMATEUR OPTIQUE A ENCOMBREMENT RÉDUIT POUR GÉNÉRER UNE PETITE TACHE D'ÉCLAIRAGE**
HANDLICHER OPTISCHER KOLLIMATOR ZUR ERZEUGUNG EINES KLEINEN BELEUCHTUNGSFLECKS
LOW-BULK OPTICAL COLLIMATOR FOR GENERATING A SMALL SPOT OF ILLUMINATION

(30) Priorité: 20.10.2015 FR 1560001
(43) Date de publication de la demande: 29.08.2018
(73) Titulaire: Maquet SAS, 45160 Ardon (FR)
(72) Inventeur: VALTEAU, Cécilia, 45160 Ardon (FR); VU THI, Minh Hong, 45160 Ardon (FR); GENEVRIERE, David, 45160 Ardon (FR)
(74) Mandataire: Prugneau, Philippe
(86) Numéro de dépôt international: PCT/FR2016/051951
(87) Numéro de publication internationale: WO 2017/068253

(56) Documents cités:
- WO-A1-2016/057580
- CN-A- 102 537 843
- US-A1- 2009 128 921
- US-A1- 2014 036 510
- US-A1- 2014 204 587

## Description

### Domaine technique

L'invention concerne un collimateur optique massique comprenant une calotte tronquée présentant une surface interne fonctionnant en réflexion total interne et ayant une petite base s'ouvrant sur un évidement traversant en forme de tronc de cône coaxial à la calotte, et au centre de laquelle est destiné à être placée une source de lumière, et ayant une grande base réfractive de telle sorte que la lumière incidente émise par la source de lumière est guidée en totalité par la surface interne vers la grande base de la calotte.

L'invention concerne aussi des dispositifs d'éclairage comprenant de tels collimateurs optiques, et en particulier les dispositifs d'éclairages opératoires utilisés notamment pour éclairer un champ opératoire médical.

### Technique antérieure

De manière connue, dans un dispositif d'éclairage pour éclairer un champ opératoire médical, un ou plusieurs systèmes optiques sont prévus dans une coupole d'éclairage pour faire converger la lumière émise par une source de lumière sur le champ opératoire pour notamment former une tache d'éclairement de dimension plus ou moins grande.

Ce système optique est généralement un collimateur standard massique qui se présente sous forme d'une calotte tronquée avec une surface interne fonctionnant en réflexion totale interne, la calotte ayant un évidement sous forme d'un tronc de cône coaxial à la calotte, la grande base du tronc de cône étant ouverte et confondue avec la petite base de la calotte. La source de lumière est placée au dessus de l'ouverture du tronc de cône, au niveau d'un fond de la calotte. Une lentille coaxiale à la calotte est agencée en dessous du tronc de cône de sorte à réfracter des faisceaux lumineux en direction du champ opératoire.

Aujourd'hui, il y a une demande du monde médical d'avoir un éclairage opératoire capable de fournir des petites taches d'éclairement, de l'ordre de quelques centimètres, pour éclairer une zone du corps où on réalise des petites incisions par exemple. Il est connu, qu'avec un collimateur standard tel que décrit ci-dessus et une LED donnée comme source de lumière, il est nécessaire d'augmenter la hauteur et le diamètre du collimateur standard pour faire une petite tache d'éclairement par rapport à un collimateur standard qui donne une grande tache d'éclairement. Le collimateur standard dit « petite tache d'éclairement » sera alors beaucoup plus encombrant que le collimateur standard dit « grande tache d'éclairement ». Or, les fabricants de dispositifs d'éclairage opératoire cherchent à diminuer le volume et le poids des coupoles d'éclairage afin de faciliter leur manipulation par du personnel médical au dessus d'un champ opératoire. Le surplus de volume et de poids apporté par un collimateur standard « petite tache d'éclairement » n'est pas compatible avec la demande de fournir une coupole d'éclairage la plus fine et légère possible.

D'autre part, les collimateurs standards massiques tels que décrits ci-dessus sont réalisés par injection plastique. Or, le cout d'une pièce réalisée par injection plastique est fortement lié à son épaisseur car elle a un impact sur le temps d'injection. Ainsi, un collimateur standard dit « petite tache d'éclairement » ayant une hauteur supérieure à un collimateur standard dit « grande tache d'éclairement », sa durée de production augmentant, le coût de fabrication augmente.

Enfin, il est connu que dans un collimateur standard, des rayons réfléchis par perte de Fresnel sur le cône coté LED passent par la lentille et prennent un chemin optique non utile pour créer un halo parasite sur le champ opératoire qui est incommodant pour le personnel médical.

De différents documents, on connait des dispositifs d'éclairage avec des collimateurs présentant des modifications par rapport à un collimateur standard massique, mais ceux-ci ne permettent pas encore de répondre correctement à la demande petite tache d'éclairement. CN 102.537.843 divulgue un collimateur optique massique.

### Exposé de l'invention

Le but de l'invention est de remédier à ces inconvénients en proposant un collimateur optique massique avec une nouvelle structure.

A cet effet, l'invention a pour objet dispositif d'éclairage opératoire formant une tache d'éclairement et comprenant une coupole d'éclairage avec plusieurs dispositifs d'éclairage, chaque dispositif d'éclairage comprenant un collimateur optique massique comprenant une calotte tronquée présentant une surface interne fonctionnant en réflexion totale interne et ayant une petite base s'ouvrant sur un évidement traversant en forme de tronc de cône coaxial à la calotte et au centre de laquelle est placée une source de lumière et ayant une grande base réfractive de telle sorte que la lumière incidente émise par la source de lumière est guidée en totalité par la surface interne vers la grande base de la calotte, l'évidement du collimateur étant disposé dans la calotte de telle sorte à présenter une petite base ouverte et confondue avec la petite base de la calotte, et en ce que les collimateurs sont orientés pour concentrer les flux lumineux des sources de lumière sur la tache d'éclairement qui est centrée dans l'axe d'éclairement de la coupole d'éclairage.

Plus particulièrement, selon l'invention :
- l'évidement du collimateur débouche dans la grande base de la calotte ;
- l'évidement comprend un premier évidement en tronc de cône avec une petite base ouverte et confondue avec la petite base de la calotte et un second évidement coaxial sous le premier évidement et entre les deux évidements un épaulement annulaire porte un écran pour la lumière incidente ;
- l'écran est absorbant coté source de lumière ;
- l'écran est diffusant coté source de lumière ;
- l'écran est collé sur l'épaulement ;
- l'épaulement forme une surface plane perpendiculaire à l'axe de la calotte ;
- le collimateur est rigide ;
- le collimateur est en plastique injecté ;
- le collimateur est en PMMA ;
- un support du collimateur comprend des moyens de fixation de l'écran pour la lumière incidente ;
- la source de lumière peut être une LED.

Avec un tel agencement, on a donc un collimateur complètement évidé axialement, l'évidement étant traversant et se présentant en partie sous forme d'un tronc de cône renversé par rapport au tronc de cône d'un collimateur massique standard. Ici le collimateur présente un trou à la place de la lentille normalement agencée dans un collimateur standard.

Avec cet agencement, dans un dispositif d'éclairage la source de lumière peut être agencée au plus près du collimateur.

Le collimateur comporte une première interface optique utile correspondant aux parois du tronc de cône renversé qui réfractent la lumière, la lumière non réfractée débouchant sur un trou du coté de la grande base du tronc de cône ; une seconde interface optique utile correspondant à la face interne de la surface externe du collimateur fonctionnant en réflexion totale interne ; une troisième interface optique utile annulaire en sortie de collimateur correspondant à la grande base de la calotte tronquée pour réfracter la lumière vers un champ opératoire pour former une tache d'éclairement.

Avec cet agencement, l'évidement traversant est une superposition de deux troncs de cône coaxiaux avec une interface ouverte, ou trou central, pouvant être masqué avec un écran pour empêcher lumière émise en direct d'atteindre le champ d'éclairage. Ainsi, il n'y a plus de halos parasites sur le champ d'éclairage.

Avec cet agencement, on obtient un collimateur pouvant former une petite tache d'éclairement présentant une hauteur réduite, donc un coût de fabrication réduit.

Le dispositif d'éclairage opératoire, comprend une coupole d'éclairage avec plusieurs dispositifs d'éclairage tels que définis plus haut.

Avec cet agencement la coupole d'éclairage du dispositif d'éclairage opératoire présente un encombrement réduit et permet de produire sur le champ opératoire une petite tache d'éclairement de 4 à 30 centimètres de diamètre lorsque la coupole d'éclairage est à une distance entre 70 et 150 centimètres du champ opératoire.

### Présentation des dessins

La présente invention sera mieux comprise et d'autres avantages apparaîtront à la lecture de la description détaillée qui suit, donnée uniquement à titre d'exemple non limitatif et illustrée par les dessins annexés, dans lesquels :
- la figure 1 est une vue schématique en perspective d'un dispositif d'éclairage opératoire selon l'invention utilisé dans un bloc opératoire ;
- la figure 2 est une vue schématique en perspective du dessous d'une coupole d'éclairage comportant des collimateurs selon l'invention ;
- la figure 3 est un schéma de principe illustrant la réflexion totale interne et la réfraction des rayons lumineux produits par une source de lumière dans un collimateur massique de l'état de l'art ;
- la figure 4 est un schéma de principe illustrant la réflexion et la réfraction des rayons lumineux produits par une source de lumière dans un collimateur massique selon l'invention ;
- la figure 5 est une vue schématique de profil d'un collimateur massique selon l'invention ;

### Description d'un exemple de réalisation

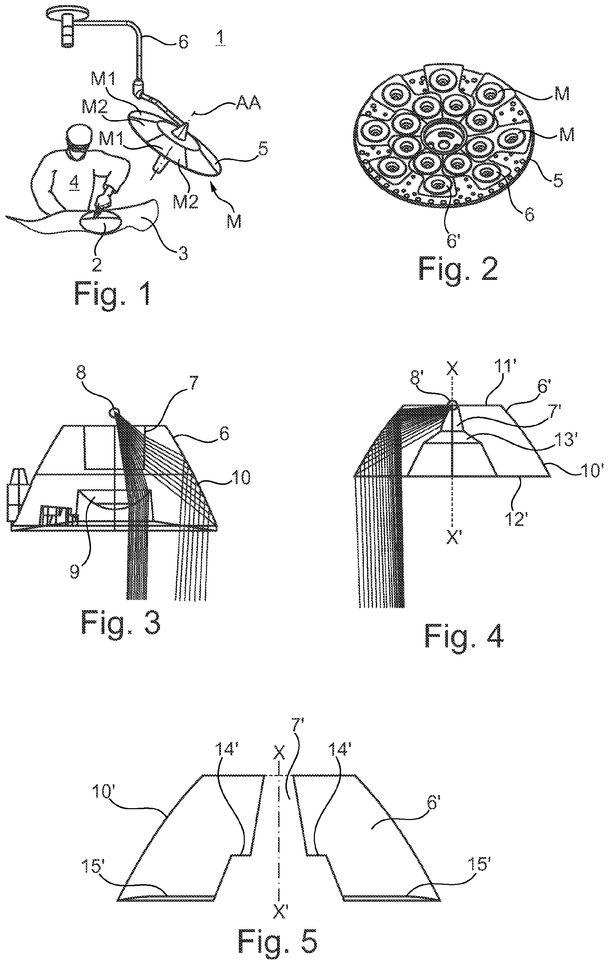

La figure 1 illustre un dispositif d'éclairage opératoire selon l'invention qui est utilisé dans un bloc opératoire pour former une tache d'éclairement 2 de petite dimension sur un champ opératoire 3, par exemple sur le corps d'un patient opéré par un chirurgien 4.

Le dispositif d'éclairage 1 présente ici une coupole d'éclairage 5 du type suspendue au plafond du bloc opératoire par l'intermédiaire d'un bras de suspension 6 articulé.

Comme illustré schématiquement sur la figure 1, la coupole d'éclairage 5 présente ici une pluralité de quartiers formant autant de modules d'éclairage M différents.

Chaque module d'éclairage tel que M1, M2 peut être pourvu d'une pluralité de sources de lumière associées respectivement à une pluralité de collimateurs qui sont orientés pour concentrer les flux lumineux des sources de lumière sur la tache d'éclairement 2 qui est centrée dans l'axe d'éclairement AA de la coupole d'éclairage 5.

La tache d'éclairement 2 est typiquement située entre 0,7 et 1,5 mètre de la coupole d'éclairage 5.

De façon générale, la tache d'éclairement 2 peut avoir un diamètre de quelques centimètres à plusieurs dizaines de centimètres.

Les sources de lumière dans les différents modules M1, M2 sont ici alimentées sélectivement et individuellement en courant par une ou plusieurs unités d'alimentation électrique couplées à une unité de contrôle/commande (non représentées). Les différentes sources de lumière peuvent donc être alimentées avec des courants ayant des intensités I différentes et variables.

La figure 2 illustre un exemple de coupole d'éclairage 5 vue de dessous sur laquelle on peut distinguer des profils de collimateurs 6, 6' issus de plusieurs modules d'éclairage M.

La figure 3 illustre un collimateur standard 6 massique selon l'état de l'art. Le collimateur standard se présente sous forme d'une calotte tronquée avec une surface interne 10 fonctionnant en réflexion totale interne. La calotte tronquée a une petite base et une grande base. Du coté de la petite base, la calotte a un premier évidement sous forme d'un tronc de cône 7 coaxial à la calotte, la grande base du tronc de cône 7 étant ouverte et confondue avec la petite base de la calotte. La petite base de l'évidement en forme de tronc de cône 7 est fermée. Les deux surfaces internes du tronc de cône 7 et la petite base du tronc de cône 7 réfractent les faisceaux de lumière incidente émis par une source de lumière 8 placée au dessus de l'ouverture du tronc de cône 7, au niveau d'un fond de la calotte.

Un second évidement 9 coaxiale à la calotte et au premier évidement est agencé en dessous du tronc de cône 7. Ce second évidement 9 présente une interface optique comme une lentille, de sorte à réfracter des faisceaux lumineux reçus en direction du champ opératoire 3.

La surface interne 10 réfléchie les faisceaux lumineux reçus, en direction du champ opératoire 3.

De manière connue, le collimateur 6 standard combine la lumière réfractée par l'interface optique 9 et la lumière réfléchie par la surface interne 10 en un point sur le champ opératoire de sorte à former une tâche d'éclairement 2.

La figure 4 illustre un collimateur 6' selon l'invention. Le collimateur 6' selon l'invention se présente ici sous forme d'une calotte tronquée, avec une surface interne 10' fonctionnant en réflexion total interne. Cette calotte s'étend suivant une direction axiale XX' entre une petite base 11' et une grande base 12' .

Une source de lumière 8', une LED par exemple, est agencée centralement par rapport à la petite base 11' de la calotte tronquée. Le collimateur 6' guide les faisceaux de lumière vers la grande base 12'.

La petite base 11' s'ouvre sur un évidement 7' traversant. Cet évidement a la forme d'un tronc de cône agencée axialement à la calotte avec une petite base ouverte et confondue avec la petite base 11' de la calotte, et une grande base orientée vers la grande base 12' de la calotte.

L'interface optique formée par les parois de l'évidement 7' réfracte la lumière émise par la source de lumière 8' vers la surface interne 10' de la calotte.

Selon l'invention, et comme visible sur les figures 4 et 5, l'évidement comprend un premier évidement en forme de tronc de cône 7' avec la petite base ouverte et confondue avec la petite base 11' de la calotte et un second évidement, ici sous forme d'un second tronc de cône, agencé coaxialement sous le premier évidement. L'évidement, comprenant les premier et second évidements, étant traversant, il débouche dans la grande base 12' de la calotte, et présente ainsi un trou en direction du champ opératoire 3. Entre les premier et second évidements, il est prévu un épaulement 14' annulaire formant une surface plane perpendiculaire à l'axe de la calotte ou un méplat, visible sur la figure 5.

Sur cet épaulement 14' peut être agencé un écran de sorte à empêcher les faisceaux lumineux de passer par le trou.

L'écran 13' peut être absorbant ou diffusant du coté source de lumière 8'. L'écran 13' peut par exemple se présenter sous forme d'une gommette ou pastille collée sur le méplat de sorte à boucher le trou. L'écran 13' peut aussi se présenter sous la forme d'un cache intégré par tous moyens de fixation connus au support du collimateur 6' dans la coupole d'éclairage 5. L'écran 13' peut aussi être bouterollé. L'écran 13' peut être clipsé.

Il est à noter qu'ici seul le premier évidement en forme de tronc de cône 7' est fonctionnel optiquement.

Il pourrait aussi y avoir plus de deux évidements agencés coaxialement.

Comme illustré sur figure 5, du fait de l'évidement traversant, la grande base 12' de la calotte du collimateur 6' massique est une interface optique annulaire 15' qui est réfractive de sorte à focaliser les faisceaux lumineux en direction du champ opératoire 3 pour former la tache d'éclairement 2.

La grande base 12' peut être plane, mais selon l'invention elle pourrait aussi être concave ou convexe. Dans certaines conditions, la grande base 12' pourrait aussi posséder une structure à micro-lentilles.

Le collimateur 6' massique selon l'invention est rigide.

Le collimateur 6' massique est fabriqué en plastique injecté, comme visible en partie sur la figure 5. Il peut être en poly(méthacrylate de méthyle) ou PMMA.

Le collimateur 6' pourrait aussi être usiné directement dans la masse de plastique.

Comme énoncé précédemment avec un collimateur standard tel qu'illustré sur la figure 3 et une LED donnée, pour faire une petite tache d'éclairement il est nécessaire d'augmenter la hauteur et le diamètre du collimateur standard par rapport à un collimateur standard qui donne une grande tache d'éclairement.

A titre d'exemple, pour une source de lumière à LED donnée, pour former une tache d'éclairement de 215 mm de diamètre avec un rendement optique de 80%, un collimateur standard doit mesurer 26 mm de diamètre et 14 mm de hauteur. Avec la même LED, pour former une tache d'éclairement de 63 mm de diamètre avec un rendement optique de 72%, le collimateur standard doit mesurer 92 mm de diamètre et 52 mm de hauteur. Donc de manière connue, pour une LED donnée, plus on veut une petite tache d'éclairement, plus les diamètres et hauteurs du collimateur sont grands.

Toujours avec la même LED, pour former une tache d'éclairement de 79 mm de diamètre, le collimateur standard doit avoir un diamètre de 85 mm et une hauteur de 48 mm, alors que pour former la même tache d'éclairement, un collimateur selon l'invention présente un diamètre de 74 mm et une hauteur de 26mm. Ainsi, pour obtenir une même tache d'éclairement il est possible d'avoir un gain de 46% sur la hauteur du collimateur en utilisant un collimateur selon l'invention. Avec un collimateur selon l'invention, il est alors possible de diminuer l'épaisseur d'une coupole d'éclairage d'un dispositif d'éclairage opératoire. De plus, la hauteur du collimateur selon l'invention étant réduit par rapport à un collimateur standard, le cout du collimateur selon l'invention réalisé en plastique injecté est donc réduit.

Dans un dispositif d'éclairage 1, chaque module d'éclairage M peut être pourvu d'une pluralité de sources de lumière 8' associées respectivement à une pluralité de collimateurs 6' selon l'invention qui sont alors orientés pour concentrer les flux lumineux des sources de lumière 8' sur la tache d'éclairement 2 qui est centrée dans l'axe d'éclairement AA de la coupole d'éclairage 5.

Selon l'invention, la petite tache d'éclairement formée a un diamètre entre 4 et 30 centimètres lorsque la coupole d'éclairage 5 est à une distance entre 70 et 150 centimètres du champ opératoire.

Dans une coupole d'éclairage 5, il est possible d'avoir un mélange de collimateurs 6 standards pour faire une grande tache d'éclairement et de collimateurs 6' selon l'invention pour faire une petite tache d'éclairement.

Selon l'invention, en pilotant séparément les courants des LEDs couplées aux collimateurs 6 standards de ceux des LEDs couplées aux collimateurs 6' selon l'invention, on peut ajuster le diamètre de la tache d'éclairement.

Dans une coupole d'éclairage 5, il serait aussi possible d'avoir un mélange de collimateurs 6' selon l'invention pour faire différentes petites taches d'éclairement, de diamètre d1 et d2, avec d1 différent de d2, en combinaison ou pas de collimateurs 6 standards pour faire une grande tache d'éclairement.

Selon l'invention, la source de lumière 8' peut être une LED, une LED COB, une diode laser couplée à un élément de phosphore, une LED multichip de couleur, ou encore une matrice de LEDs.

Selon l'invention, la source de lumière 8' peut être agencée au plus près du collimateur 6'. Les rayons proches de l'axe seront perdus, entrainant une perte d'énergie lumineuse. Néanmoins, cette perte d'énergie lumineuse n'est pas très importante car pour former une petite tache d'éclairement, moins d'énergie lumineuse est demandée.

Il va de soi que la présente invention ne saurait être limitée à la description qui précède de certains modes de réalisation, susceptibles de subir quelques modifications sans pour autant sortir du cadre de l'invention.

## Revendications

1. Dispositif d'éclairage opératoire formant une tache d'éclairement (2) et comprenant une coupole d'éclairage (5) avec plusieurs dispositifs d'éclairage (1), chaque dispositif d'éclairage (1) comprenant un collimateur (6') optique massique comprenant une calotte tronquée présentant une surface interne (10') fonctionnant en réflexion totale interne et ayant une petite base (11') s'ouvrant sur un évidement traversant en forme de tronc de cône (7') coaxial à ladite calotte et au centre de laquelle est placée une source de lumière (8') et ayant une grande base (12') réfractive de telle sorte que une lumière incidente émise par ladite source de lumière (8') est guidée en totalité par ladite surface interne (10') vers ladite grande base (12') de ladite calotte, ledit évidement dudit collimateur (6') étant disposé dans ladite calotte de telle sorte à présenter une petite base ouverte et confondue avec ladite petite base de ladite calotte, lesdits collimateurs (6') étant orientés pour concentrer les flux lumineux desdites sources de lumière (8') sur ladite tache d'éclairement (2) qui est centrée dans l'axe d'éclairement (AA) de ladite coupole d'éclairage (5).

2. Dispositif d'éclairage opératoire selon la revendication 1, **caractérisé en ce que** ledit évidement dudit collimateur (6') débouche dans ladite grande base de ladite calotte.

3. Dispositif d'éclairage opératoire selon la revendication 2, **caractérisé en ce que** ledit évidement comprend un premier évidement en tronc de cône (7') avec une petite base ouverte et confondue avec ladite petite base de ladite calotte et un second évidement coaxial sous ledit premier évidement et entre lesdits deux évidements un épaulement (14') annulaire porte un écran (13') pour ladite lumière incidente.

4. Dispositif d'éclairage opératoire selon la revendication 3, **caractérisé en ce que** ledit écran (13') est absorbant coté source de lumière (8').

5. Dispositif d'éclairage opératoire selon la revendication 3, **caractérisé en ce que** ledit écran (13') est diffusant coté source de lumière (8').

6. Dispositif d'éclairage opératoire selon les revendications 3 à 5, **caractérisé en ce que** ledit écran (13') est collé sur ledit épaulement (14').

7. Dispositif d'éclairage opératoire selon la revendication 3, **caractérisé en ce que** ledit épaulement (14') forme une surface plane perpendiculaire à l'axe de ladite calotte.

8. Dispositif d'éclairage opératoire selon l'une des revendications 1 à 7, **caractérisé en ce que** ledit collimateur (6') est rigide.

9. Dispositif d'éclairage opératoire selon l'une des revendications 1 à 8, **caractérisé en ce que** ledit collimateur (6') est en plastique injecté.

10. Dispositif d'éclairage opératoire selon l'une des revendications 1 à 9, **caractérisé en ce que** ledit collimateur (6') est en PMMA.

11. Dispositif d'éclairage opératoire selon la revendication 3, **caractérisé en ce qu'**un support du collimateur comprend des moyens de fixation dudit écran (13') pour ladite lumière incidente.

12. Dispositif d'éclairage opératoire selon l'une des revendications 1 à 11, **caractérisé en ce que** ladite source de lumière (8') est une LED.

## Patentansprüche

1. Operationsbeleuchtungsvorrichtung, die einen Beleuchtungsfleck (2) bildet und eine Beleuchtungskuppel (5) mit mehreren Beleuchtungsvorrichtungen (1) umfasst, wobei jede Beleuchtungsvorrichtung (1) einen optischen Massenkollimator (6') umfasst, mit einer abgeschnittenen Kalotte, die eine Innenfläche (10') aufweist, die in vollständiger innerer Reflexion funktioniert und die eine kleine Basis (11') aufweist, die sich auf einer zu der Kalotte koaxialen durchgehenden Ausnehmung in Gestalt eines Kegelstumpfes (7') öffnet und in deren Mitte eine Lichtquelle (8') angeordnet ist, und die eine lichtableitende große Basis (12') derart aufweist, dass ein von der Lichtquelle (8') ausgesandtes auftreffendes Licht vollständig durch die innere Oberfläche (10') zu der großen Basis (12') der Kalotte geführt wird,
wobei die Ausnehmung des Kollimators (6') in der Kalotte derart angeordnet ist, dass diese eine offene und mit der kleinen Basis der Kalotte zusammenfallende kleine Basis aufweist,
wobei die Kollimatoren (6') orientiert sind zum Konzentrieren des Lichtstroms der Lichtquellen (8') auf den Beleuchtungsfleck (2), der in der Beleuchtungsachse (AA) der Beleuchtungskuppel (5) zentriert ist.

2. Operationsbeleuchtungsvorrichtung nach Anspruch 1, **dadurch gekennzeichnet, dass** die Aussparung des Kollimators (6') in der großen Basis der Kalotte mündet.

3. Operationsbeleuchtungsvorrichtung nach Anspruch 2, **dadurch gekennzeichnet, dass** die Ausnehmung eine erste kegelstumpfförmige Ausnehmung (7') mit einer offenen und mit der kleinen Basis der Kalotte zusammenfallenden kleinen Basis und eine koaxiale zweite Ausnehmung unterhalb der ersten Ausnehmung sowie zwischen den zwei Ausnehmungen einen ringförmigen Absatz (14'), der eine Abschirmung (13') für das inzidente Licht trägt, umfasst.

4. Operationsbeleuchtungsvorrichtung nach Anspruch 3, **dadurch gekennzeichnet, dass** die Abschirmung (13') auf der Seite der Lichtquelle (8') absorbierend ist.

5. Operationsbeleuchtungsvorrichtung nach Anspruch 3, **dadurch gekennzeichnet, dass** die Abschirmung (13') auf der Seite der Lichtquelle (8') streuend ist.

6. Operationsbeleuchtungsvorrichtung nach den Ansprüchen 3 bis 5, **dadurch gekennzeichnet, dass** die Abschirmung (13') auf den Absatz (14') geklebt ist.

7. Operationsbeleuchtungsvorrichtung nach Anspruch 3, **dadurch gekennzeichnet, dass** der Absatz (14') eine zu der Achse der Kalotte senkrechte flache Fläche bildet.

8. Operationsbeleuchtungsvorrichtung nach einem der Ansprüche 1 bis 7, **dadurch gekennzeichnet, dass** der Kollimator (6') steif ist.

9. Operationsbeleuchtungsvorrichtung nach einem der Ansprüche 1 bis 8, **dadurch gekennzeichnet, dass** der Kollimator (6') aus gespritztem Kunststoff ist.

10. Operationsbeleuchtungsvorrichtung nach einem der Ansprüche 1 bis 9, **dadurch gekennzeichnet, dass** der Kollimator (6') aus PMMA ist.

11. Operationsbeleuchtungsvorrichtung nach Anspruch 3, **dadurch gekennzeichnet, dass** ein Träger des Kollimators Befestigungsmittel der Abschirmung (13') für das auftreffende Licht umfasst.

12. Operationsbeleuchtungsvorrichtung nach einem der Ansprüche 1 bis 11, **dadurch gekennzeichnet, dass** die Lichtquelle (8') eine LED ist.

## Claims

1. A surgical lighting device forming an illumination spot (2) and comprising a lighting dome (5) having a plurality of lighting devices (1), each lighting device (1) comprising a solid optical collimator (6') comprising a truncated cap having an inside surface (10') operating in total internal reflection and having a small base (11') opening into a frustoconical through recess (7') that is coaxial with said cap and at the centre of which a light source (8') is placed, and having a refractive large base (12') such that incident light emitted by said light source (8') is guided in totality by said inside surface (10') towards said large base (12') of said cap, said recess of said collimator (6') being disposed in said cap in such a manner as to have a small base open and coinciding with said small base of said cap, and said collimators (6') being directed to concentrate the light flux from said light sources (8') onto said illumination spot (2) that is centered on the illumination axis (AA) of said lighting dome (5).

2. A surgical lighting device according to claim 1, **characterized in that** said recess in said collimator (6') opens out into said large base of said cap.

3. A surgical lighting device according to claim 2, **characterized in that** said recess comprises a frustoconical first recess (7') having a small base open and coinciding with said small base of said cap, and a second coaxial recess under said first recess and, between the two recesses, an annular shoulder (14') carries a screen (13') for said incident light.

4. A surgical lighting device according to claim 3, **characterized in that** said screen (13') is absorbent on its side closer to the light source (8').

5. A surgical lighting device according to claim 3, **characterized in that** said screen (13') is diffusive on its side closer to the light source (8').

6. A surgical lighting device according to claims 3 to 5, **characterized in that** said screen (13') is adhesively bonded to said shoulder (14').

7. A surgical lighting device according to claim 3, **characterized in that** said shoulder (14') forms a plane surface that is perpendicular to the axis of said cap.

8. A surgical lighting device according to any one of claims 1 to 7, **characterized in that** said collimator (6') is rigid.

9. A surgical lighting device according to any one of claims 1 to 8, **characterized in that** said collimator (6') is made of injection-molded plastic.

10. A surgical lighting device according to any one of claims 1 to 9, **characterized in that** said collimator (6') is made of PMMA.

11. A surgical lighting device according to claim 3, **characterized in that** a support of the collimator includes means for fastening said screen (13') for said incident light.

12. A surgical lighting device according to any one of claims 1 to 11, **characterized in that** said light source (8') is an LED.
